# EUROPEAN PATENT SPECIFICATION

(11) **EP 3 532 024 B1**
(45) Date of publication and mention of the grant of the patent: **24.02.2021**
(21) Application number: 17797559.6
(22) Date of filing: 27.10.2017
(51) Int. Cl.: A61K 8/64, C07K 14/47, C07K 7/00, A61Q 19/08, C07K 7/06

(54) **COSMETIC USE AND METHOD FOR IMPROVING PHYSIOLOGICAL FUNCTIONS OF THE SKIN BY INCREASING THE EXPRESSION OF UBIAD1**
KOSMETISCHE VERWENDUNG UND VERFAHREN ZUR VERBESSERUNG DER PHYSIOLOGISCHEN FUNKTIONEN DER HAUT DURCH ERHÖHUNG DER EXPRESSION VON UBIAD1
UTILISATION COSMÉTIQUE ET PROCÉDÉ D'AMÉLIORATION DES FONCTIONS PHYSIOLOGIQUES DE LA PEAU EN AUGMENTANT L'EXPRESSION D'UBIAD1

(30) Priority: 28.10.2016 CN 201610968180
(43) Date of publication of application: 04.09.2019
(73) Proprietor: ISP Investments LLC, Wilmington, Delaware 19805 (US)
(72) Inventor: CUCUMEL, Karine, 06650 Opio (FR); GONDRAN, Catherine, 83440 Callian (FR); LABARRADE, Florian, 06600 Antibes (FR)
(74) Representative: Miquel, Corinne
(86) International application number: PCT/EP2017/077565
(87) International publication number: WO 2018/078079

(56) References cited:
- WO-A1-2012/107073
- FR-A1- 2 915 395
- US-A1- 2014 163 118
- VERA MUGONI ET AL: "Ubiad1 Is an Antioxidant Enzyme that Regulates eNOS Activity by CoQ10 Synthesis", CELL, vol. 152, no. 3, 1 January 2013 (2013-01-01), pages 504-518, XP055436735, US ISSN: 0092-8674, DOI: 10.1016/j.cell.2013.01.013

## Description

### Field of the invention

This invention relates to a cosmetic use of a composition comprising a peptide of formula (I) or its derivative for improving physiological functions of the skin, comprising the step of contacting skin, skin appendages or epidermal cells including keratinocytes with said composition and increasing the expression of Ubiad1 in such skin, skin appendages or epidermal cells. This invention also relates to a method of increasing the expression of Ubiad1. More specifically, the invention relates to a method of increasing the expression of Ubiad1 by using the peptide of formula (I).

### Background

The skin is a covering organ comprised of epidermis, dermoepidermal junction, dermis, etc. The outermost part is the epidermis, a multistratified epithelium consisting essentially of keratinocytes closely linked to one another. The basal layer of the epidermis is comprised of a layer of proliferative cells, primarily keratinocytes and melanocytes, which are anchored on the dermoepidermal junction. The dermis is the tissue supporting the skin and is comprised of water, elastin fibers and collagen fibers (70% of dermal fibers), enveloped in an interstitial matrix of proteoglycans. Fibroblasts are the main cellular component of the dermis and are the source of collagen fiber and elastin fiber synthesis.

Chronological aging will cause the decrease of intrinsic anti-oxidation and repairment of skin. In addition, human skin is exposed to environmental stresses including UV radiation, air pollution and chemical toxicants, which will cause skin aging.

People have developed many products in order to improve the physiological functions of the skin, including the products capable of activating cell regeneration, reinforcing extracellular collagen and elastin matrix, and increasing the levels of Vitamin E, CoQ10 and ascorbic acid. However, the identification of new mechanisms/targets and compositions with respect to the care of the skin is still important to the development of the related products (WO2012/107073, FR2915395).

### Description of the Invention

The invention relates to a cosmetic us of a composition comprising the peptide of formula (I) or the derivative thereof,

R₁-(AA)ₙ-Ala-Val-Leu-Ala-Gly-(AA)ₚ-R₂ (I)

wherein
AA represents any amino acid or one of its derivatives, and n and p are integers between 0 and 4;
R₁ represents the primary amine function of the N-terminal amino acid, free or substituted by a protector group which can be selected from acetyl, benzyl, tosyl and benzyloxycarbonyl;
R₂ represents the hydroxyl function of the carboxyl acid of C-terminal amino acid, free or substituted by a protector group which can be selected from C₁ to C₃₀ alkyl, -NH₂, -NHY and -NYY group with Y representing a C₁ to C₄ alkyl,
for improving physiological functions of the skin, comprising the step of contacting skin, skin appendages or epidermal cells including keratinocytes with said composition and increasing the expression of Ubiad1 in such skin, skin appendages or epidermal cells. The scope of the invention is defined by the appended claims.

The peptide of formula (I) consists of the amino acid sequence of SEQ ID NO:1 or SEQ ID NO:2, preferably SEQ ID NO:2.

In one embodiment, the cells are keratinocytes, preferably epidermal keratinocytes.

In one embodiment, skins are organs including skin and skin appendages.

In one embodiment, the skin is preferably epidermis tissues, more preferably spinous layer and basal layer of the epidermis.

The composition of the present invention can increase the expression of Ubiad1, which can improve the physiological functions of the skin, including for example protection from oxidative stress and lipid peroxidation, and preservation of cell membrane fluidity/permeability. The present invention has a good application prospect in skin care.

The present invention is directed to the cosmetic use of the peptides as defined in claim 1 for increasing the expression of Ubiad1 in organs, tissues or cells, *i.e.* skin, skin appendages or epidermal cells including keratinocytes for improving physiological functions of the skin.

### Brief Description of the Figures

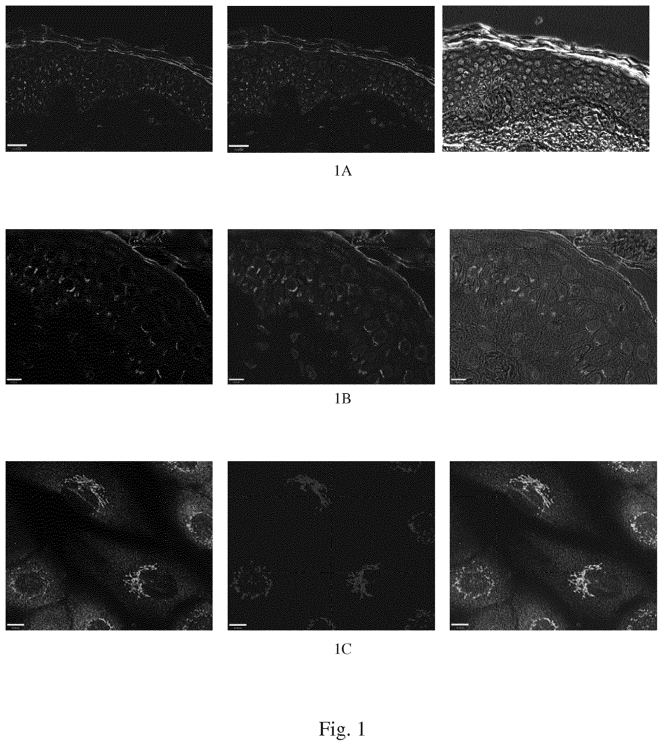

Figure 1: Expression of Ubiad1 protein in normal skin.
   (A) Immunodetection of Ubiad1 (green) and DNA stained with DAPI (blue), Ubiad1 is positive in the epidermal spinous and basal layers (x20 objective lens, scale bar = 31 µm).
   (B) Immunodetection of Ubiad1 (green) and DNA stained with DAPI (blue), Ubiad1 appears to a tight perinuclear localization, polarly confined (organelle-like) (x63 objective lens, scale bar = 10 µm).
   (C) Immunodetection of Ubiad1 (green), Giantin (red) and DNA stained with DAPI (blue), protein co-localization (yellow) in cultured human keratinocytes, Pearson's correlation coefficient for cells in middle of the field is relatively high 0.79 and 0.77 (x63 objective lens, scale bar = 10 µm).

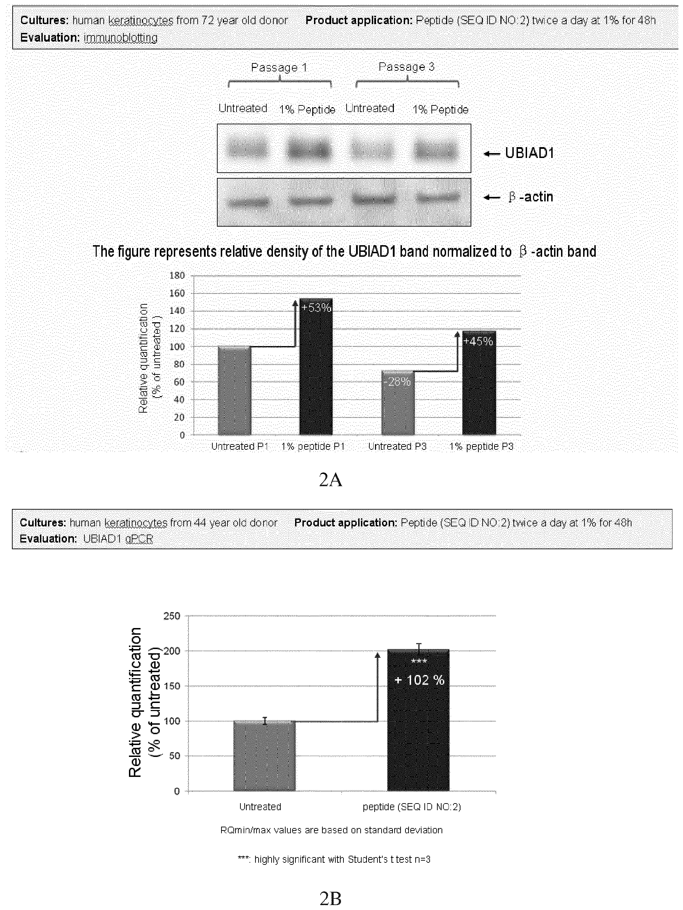

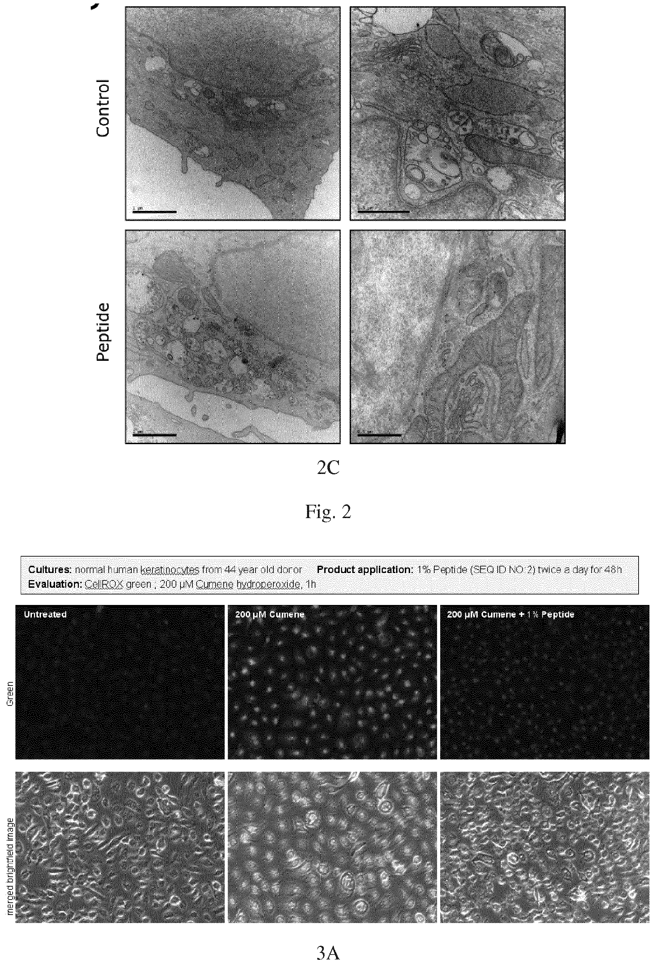

Figure 2: The increased expression of Ubiad1 in human keratinocytes by Ubiad1 inducer peptide and the implications.
   (A) Immunoblotting of Ubiad1 after successive keratinocyte culture passages (E means early passage, L means late passage), and immunoblotting of Ubiad1 after treatment with 1 µM (1%) the Ubiad1 inducer peptide.
   (B) qPCR of Ubiad1 after treatment with 1 µM (1%) the Ubiad1 inducer peptide.
   (C) Representative electron microscopy images of keratinocyte treated with the Ubiad1 peptide inducer and double-stained with uranyl acetate and lead citrate (left pictures scale bar = 1 µm; right pictures scale bar = 0.5 µm).

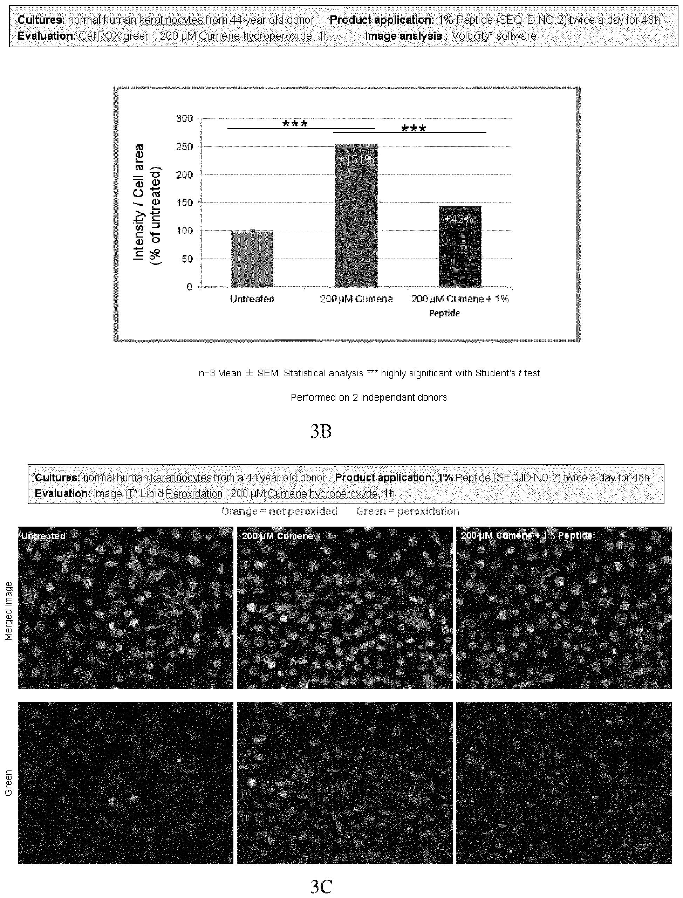

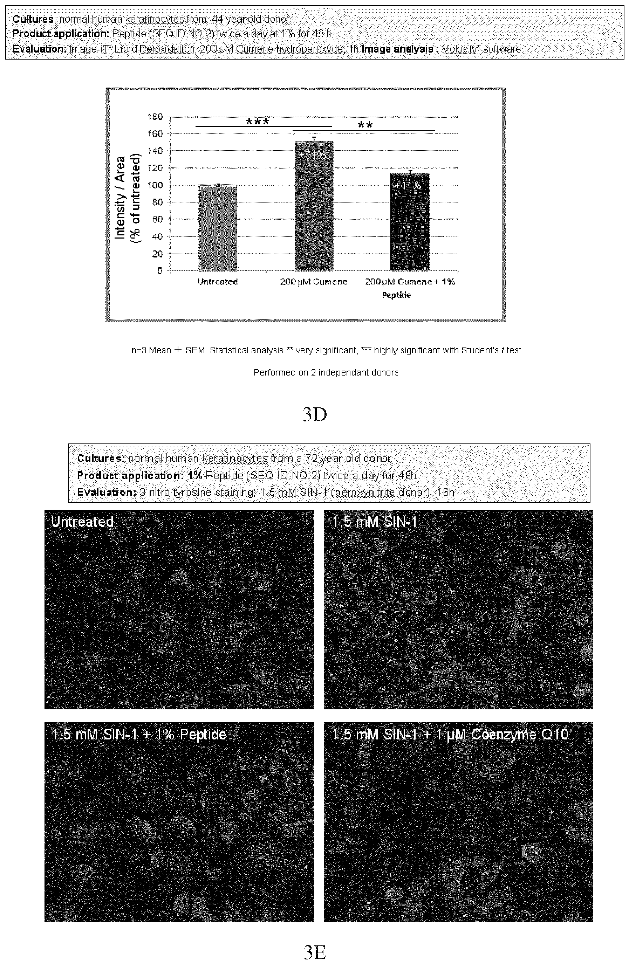

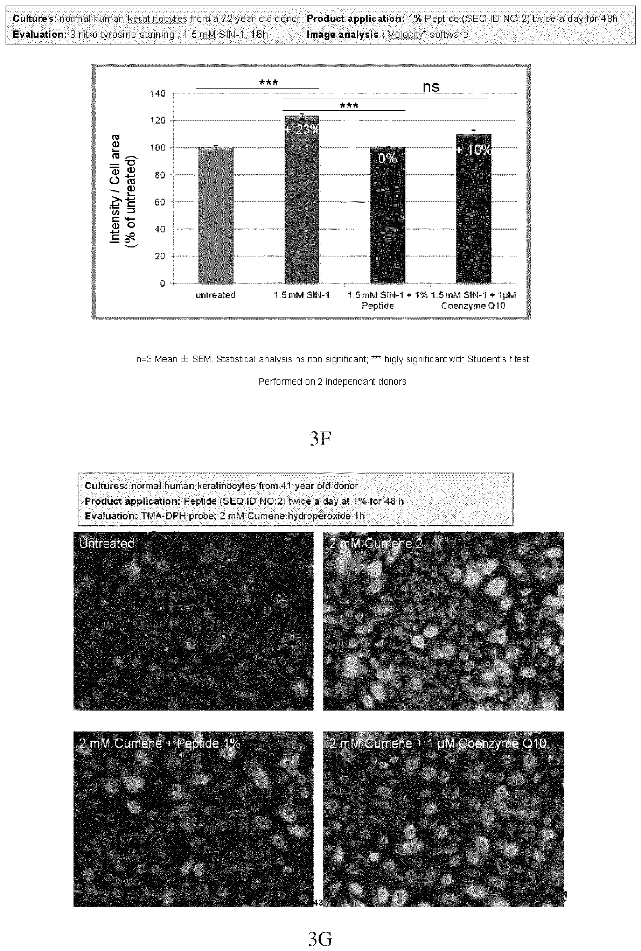

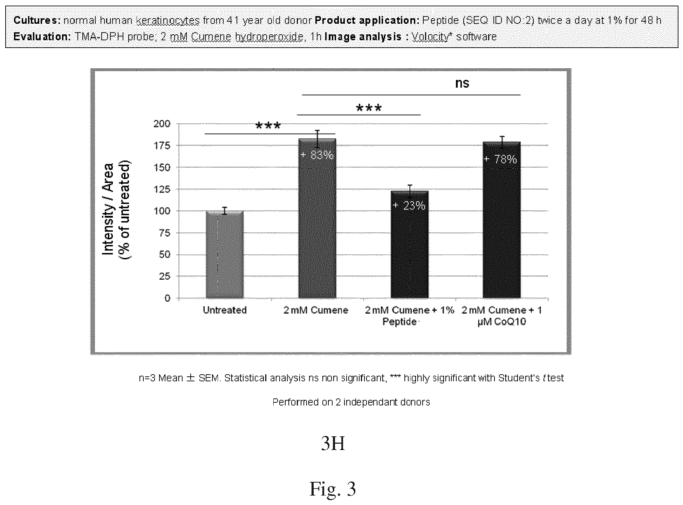

Figure 3: Protection effect of the increased expression of Ubiad1 on keratinocytes
   (A) Cellular ROS measurement in keratinocyte exposed 1 hour to 200 µM cumene hydroperoxide using the CellROX green assay (x20 objective lens, scale bar = 31 µm).
   (B) CellROX quantification graph with One-Way Analysis of Variance, displaying confidence interval means diamonds (green). The line across each diamond represents the group mean. The vertical span of each diamond represents the 95% confidence interval for each group. Grand sample mean is represented by a horizontal black line.
   (C) Lipid peroxidation in keratinocyte exposed 1 hour to 200 µM cumene hydroperoxide (reduced BODIPY in red; oxidized BODIPY in green) (x20 objective lens, scale bar = 31 µm).
   (D) Lipid peroxidation quantification graph with One-Way Analysis of Variance, displaying confidence interval means diamonds (green). The line across each diamond represents the group mean. The vertical span of each diamond represents the 95% confidence interval for each group. Grand sample mean is represented by a horizontal black line.
   (E) Immunodetection of 3-nitrotyrosine (green), F-actin staining with phalloidin (red) and DNA stained with DAPI (blue) in keratinocyte submitted to reactive nitric species stress (x20 objective lens, scale bar = 31 µm).
   (F) 3 NT quantification graph with One-Way Analysis of Variance, displaying confidence interval means diamonds (green). The line across each diamond represents the group mean. The vertical span of each diamond represents the 95% confidence interval for each group. Grand sample mean is represented by a horizontal sblack line.
   (G) Plasma membrane integrity measurement with TMA-DPH probe (blue) in keratinocyte exposed 1 hour to 2 mM cumene hydroperoxide (x20 objective lens, scale bar = 31 µm).
   (H) TMA-DPH quantification graph with One-Way Analysis of Variance, displaying confidence interval means diamonds (green). The line across each diamond represents the group mean. The vertical span of each diamond represents the 95% confidence interval for each group. Grand sample mean is represented by a horizontal black line.

### Detailed Description

Ubiad1 (also known as Tere1) was cloned in 2001 (McGarvey TW, et al., Oncogene 2001;20:1042-51). The inventors of the present invention found that Ubiad1 is located in keratinocytes of the epidermis. Light microscopic imaging of Ubiad1 and Giantin (a Golgin family protein) co-localization defined the Golgi domains, while the larger network of Ubiad1 staining pointed to the mitochondrial network. The inventors found that the increased expression of Ubiad1 is companied by the significant improvement in the ultrastructural profile of keratinocyte mitochondria and the Golgi complex. A positive effect on the mitochondrial components noted above is beneficial for cellular energetics of keratinocytes. Oxidative damage to mitochondrial DNA and proteins in keratinocytes relates to the decreased physiological functions of the skin, such as aging.

Moreover, the inventors further found that Ubiad1 plays important roles in preventing skin cells from such as oxidative stress and lipid peroxidation. The increased expression of Ubiad1 in skin cells such as keratinocytes can effectively decrease the oxidative stress and lipid peroxidation. In addition, the increased expression of Ubiad1 also helps the preservation of cell membrane fluidity/permeability.

The term "peptide" refers to a chain of two or more amino acids bound to one another by peptide bonds. The term "the peptide of the present invention" refers to the peptide of formula (I) having seq. ID No. 1 and 2. The peptide of the present invention can be obtained either by classic chemical synthesis (in the solid phase or in the liquid homogeneous phase), or by enzymatic synthesis (Kullman et al. J. Biol. Chem., 1980, vol. 225, p. 8234) from constituent amino acids. In one embodiment, the peptide of the present invention can be of natural or synthetic origin.

To improve resistance to degradation, it may be necessary to modify the peptide so as to obtain a protected form of the peptide of the present invention. The modification should be performed in a manner of a physiologically acceptable or a cosmetically acceptable form. In one embodiment, the primary amine function of the N-terminal amino acid of the peptide of the present invention can be subject to an acylation or an acetylation. Preferably, the protector group of the N-terminal amino acid can be selected from acetyl, benzyl, tosyl and benzyloxycarbonyl. In one embodiment, the hydroxyl function of the C-terminal amino acid of the peptide of the present invention can be subject to an amidation or an esterification. Preferably, the protector group of the C-terminal amino acid can be selected from C₁ to C₃₀ alkyl, -NH₂, -NHY and -NYY group with Y representing a C₁ to C₄ alkyl.

The peptide of the present invention consists of the amino acid sequence of SEQ ID NO:1 or SEQ ID NO:2, preferably SEQ ID NO:2.

In one embodiment, the organ is skin. The term "skin" includes constantly skin and "skin appendages". Skin appendages include all keratin annexes present on the surface of the body, in particular the hair, the lashes, the eyebrows, the nails and hair. The skin comprises epidermis, dermoepidermal junction, dermis, etc. The epidermis further comprises a stratum corneum, a stratum granulosum, a spinous layer and a basal layer. In one embodiment, the tissues are epidermis tissues, preferably spinous layer and basal layer of the epidermis. In one embodiment, the cells are keratinocytes, preferably epidermal keratinocytes.

The invention relates to the cosmetic use of the peptide of formula (I) for increasing the expression of Ubiad1 in keratinocytes for improving physiological functions of the skin,

R₁-(AA)ₙ-Ala-Val-Leu-Ala-Gly-(AA)ₚ-R₂ (I)

wherein
AA represents any amino acid or one of its derivatives, and n and p are integers between 0 and 4;
R₁ represents the primary amine function of the N-terminal amino acid, free or substituted by a protector group which can be selected from acetyl, benzyl, tosyl and benzyloxycarbonyl;
R₂ represents the hydroxyl function of the carboxyl acid of C-terminal amino acid, free or substituted by a protector group which can be selected from C₁ to C₃₀ alkyl, -NH₂, -NHY and -NYY group with Y representing a C₁ to C₄ alkyl, wherein the peptide of formula (I) comprises or consists of the following amino acid sequences: SEQ ID NO:1 or SEQ ID NO:2, preferably SEQ ID NO:2.

The peptide of the present invention is advantageously solubilized in one or more physiologically or cosmetically acceptable solvents, such as water, glycerol, ethanol, propylene glycol, butylene glycol, dipropylene glycol, ethoxylated or propoxylated diglycols, cyclic polyols, or any mixture of these solvents. The term "physiologically or cosmetically acceptable" means that the solvent chosen is suitable for coming into contact with the skin without causing toxicity or intolerance reactions. In one embodiment, the peptide of the present invention can be dissolved in one or more cosmetically acceptable solvents selected from water, glycerol, ethanol, propylene glycol, butylene glycol, dipropylene glycol, ethoxylated or propoxylated diglycols, cyclic polyols, or any mixture of these solvents.

The peptide of the present invention can be encapsulated or contained in a cosmetic vector such as liposomes or any other microcapsule used in the cosmetic field or adsorbed on powdery organic polymers, mineral carriers such as talcs and bentonites.

The invention also relates to use of the peptide of formula (I) as an active ingredient for increasing the expression of Ubiad1 in skin keratinocytes in a cosmetic care composition. The invention also relates to a method of cosmetic care to increase the expression of Ubiad1 in skin keratinocytes, comprising administrating a cosmetic care composition comprising the peptide of formula (I) to the skin.

The composition of the present invention may in particular be in the form of an aqueous, hydro-alcoholic or oily solution; an oil-in-water or a water-in-oil emulsion or multiple emulsions; aqueous or anhydrous gel; colloid. These compositions can also be in the form of creams, suspensions, or powders, suitable for application on the skin, mucous membranes, lips and/or skin appendages. These compositions may be more or less fluid and have the appearance of a cream, a lotion, a milk, a serum, a pomade, a cream, a paste or a foam. They may also be in solid form, such as a stick, or be applied to the skin in aerosol form. In one embodiment, the composition of the present invention is a cosmetic care composition.

The composition of the present invention include any additive commonly used in the cosmetic field as well as the adjuvant necessary for their formulation, such as co-solvents (ethanol, glycerol, benzyl alcohol, humectant, etc.), thickening agents, diluents, emulsifiers, antioxidants, coloring agents, sunscreens, pigments, fillers, preservatives, perfumes, odor absorbents, essential oils, trace elements, essential fatty acids, surfactants, film-forming polymers, chemical or mineral filters, hydrating agents or thermal water, and so on. It is possible, for example, to cite water-soluble polymers of a natural type, such as polysaccharides, or polypeptides, cellulosic derivatives of the methylcellulose or hydroxypropyl cellulose type, or synthetic polymers, poloxamers, carbomers, siloxanes, PVA or PVP, and in particular polymers sold by the ISP company.

In any case, a person skilled in the art will make sure that these adjuvants as well as their proportions are chosen so as not to counteract the advantageous properties sought in the composition according to the invention. These adjuvants may, for example, be present in concentrations ranging from 0.01 to 20% of the total weight of the composition. When the composition of the invention is an emulsion, the fatty phase may represent 5 to 80% by weight and preferably 5 to 50% by weight with respect to the total weight of the composition. The emulsifiers and co-emulsifiers used in the composition will be chosen from those conventionally used in the field considered. For example, they can be used in a proportion ranging from 0.3 to 30% by weight, with respect to the total weight of the composition.

In one embodiment, the peptide of the present invention is present in the composition in a concentration between 0.0005-500 µM, preferably 0.01-5 µM, based on the total weight of the composition.

Of course, the peptide of the present invention can be used alone or in association with other active agents. For example, the cosmetic care composition of the present invention contains, in addition, at least one other active agent intended to improve physiological functions of the skin, such as regenerating, anti-aging, anti-wrinkle, thickening, anti-free radical, anti-glycation, hydrating, antibacterial, antifungal, keratolytic, muscle relaxing, exfoliating, and toning agents, agents stimulating the synthesis of dermal macromolecules or energy metabolism, agents modulating cutaneous differentiation, pigmentation or depigmentation, agents stimulating nail or hair growth, agents stimulating microcirculation, sunscreens or metalloproteinase inhibiting agents. In a particular embodiment of the invention, the increased expression of Ubiad1 gene in skin keratinocytes can effectively result in the decrease of oxidative stress and lipid peroxidation in the skin keratinocytes, which is helpful for the prevention of skin aging.

In one embodiment, the composition of the present invention will comprise, in addition to the peptide of the present invention:
- sunscreens, ultraviolet and Infrared screens
- anti-free radical agents,
- DHEA (dehydroepiandrosterone),
- at least one cytochrome c-activating compound, and/or;
- one (or more) aquaporin-activating compound and/or;
- one (or more) sirtuin-activating compound and/or;
- one (or more) compound that increases cell adhesion and/or;
- one (or more) compound that increases the production of matrix proteins of the collagen or laminin type, etc.;
- one (or more) HSP protein-modulating compound;
- one (or more) compound that increases cell energy;
- one (or more) pigmentation-modulating compound such as a yeast, amaranth, linseed, bean, cacao, corn, soy, sunflower, rapeseed or pea peptide extract;
- one (or more) compound improving the skin barrier function;
- one (or more) mitochondria-protecting compound.
- vitamin A and notably retinoic acid, retinol, retinol proprionate, retinol palmitate,
- vitamin B3 and notably niacinamide, niconitate of tocopherol,
- vitamin B5, vitamin B6, vitamin B12, panthenol,
- vitamin C, and notably ascorbic acid, ascorbyl glucoside, ascorbyl tetrapalmitate, magnesium and sodium ascorbyl phosphate,
- vitamins E, F, H, K, PP, and coenzyme Q10,
- metalloproteinase inhibitor, activator of Tissue Inhibitor Metalloproteinase (TIMP),
- aminoacids and notably arginine, ornithine, hydroxyproline, hydroxyproline dipalmitate, palmitoylglycine, hydroxylysine, methionine and its derivatives, N-acylated aminoacids,
- natural or synthetic peptides, including, di-, tri-, tetra-, penta- and hexapeptides and their lipophilic derivatives, isomers and complex with other molecules such as metallic ion (i.e. copper, zinc, manganese, magnesium, and others), peptides sold under commercial names MATRIXYL®, ARGIRELINE®, COLLAXYL™, PEPTIDE VINCI 02™, CHRONOGEN™, LAMINIXYL IS™,
- peptidic plant extracts obtained by hydrolysis or any other methods such as soy extract, einkorn extract, vitis vinifera extract, rapeseed extract, flaxseed extract rice extract, corn extract, or pea extract, carob extract, bean extract, fava extract,
- yeast extract, artemia salina extract,
- dehydroacetic acid (DHA),
- natural or synthetic phystosterols,
- alpha- and beta-hydroxyacids, silanols,
- sugar amines, glucosamine, D-glucosamine, N-acetyl- glucosamine, N-acetyl-D-glucosamine, mannosamine, N-acetyl mannosamine, galactosamine, N-acetyl galactosamine,
- polyphenols, isoflavones, flavonoids, such as grap extract, pine extract, olive extract,
- lipids such as ceramides or phospholipids,
- animal oils such as squalenes or squalanes,
- vegetal oils, such as almond oil, coconut oil, castor oil, jojoba oil, olive oil, rapeseed oil, peanut oil, sunflower oil, wheat germ oil, corn germ oil, soybean oil, cotton oil, alfalfa oil, poppy oil, pumpkin seed oil, evening primrose oil, millet oil, barley oil, rye oil, safflower oil, passion oil, hazelnut oil, palm oil, apricot kernel oil, avocado oil, calendula oil, ethoxylated vegetable oils, or shea butter. The abovementioned compounds can be natural, such as peptide hydrolysates of plants, or also synthetic, such as peptide compounds.

The term "contacting" refers to applying the peptide of the present invention or a composition including the same to the surface of an organ or tissue including keratinocytes, *i.e.* skin, skin appendages or epidermal cells including keratinocytes. The peptide of the present invention or a composition including the same can be topically applied in a subject. The term "subject" refers to mammal, comprising but not limited to human. "Topical application" refers to the application or spreading of the peptide of the present invention, or a composition containing it, on the surface of the skin or a mucus membrane. In one embodiment, the peptide of the present invention or the composition containing it is applied to the parts of human body exposed to environments, including but not limited to head, face, head-and-neck, forehead, eyepit, nose, neck, arm and leg.

The term "improved physiological functions of the skin" means all modifications of the exterior appearance of the skin and the skin appendages, for example, the improvement of skin signs of aging and photo-aging. "Skin signs of aging and photo-aging" refers to all changes in the external appearance of the skin and skin appendages due to aging, such as, for example, thinning of the skin, sagging, loss of hydration and atonia, deep wrinkles and fine lines, loss of firmness and tone, dermal atrophy, loss of skin tone homogenization, dark circle, or any other internal degradation of the skin resulting from exposure to ultraviolet radiation, liver spots and age spots. Liver spots also known as "Solar lentigo", "Lentigo senilis", "Old age spot", "Senile freckle", are blemishes on the skin associated with aging and photo-aging due to exposure to ultraviolet radiation from the sun. They range in color from light brown to red or black and are located in areas most often exposed to the sun, particularly the hands, face, shoulders, arms and forehead, and the scalp if bald. In a particular embodiment of the invention, the increased expression of Ubiad1 gene in skin keratinocytes can result in the above improvements, including for example the improvements of skin signs of aging and photo-aging, the decrease of skin wrinkles, dark circle, etc.

Other advantages and features of the invention will become clearer in view of the following examples provided for illustrative and non-limiting purposes.

### Examples

### 1. Materials & Methods

### 1.1 Structure of Ubiad1 inducer peptide

The amino acid sequence of Ubiad1 inducer peptide is Ala-Val-Leu-Ala-Gly-NH2 (SEQ ID NO: 2), and the Ubiad1 inducer peptide is artificially synthesized. The working solution was 1 µM.

### 1.2 Antibodies and probes

Primary antibodies used were anti-Ubiadl (sc-377013, Santa Cruz Biotechnology, Heidelberg, Germany), anti-Giantin (ab24586, Abcam, Cambridge, UK), anti-3-Nitrotyrosine (ab61392, Abcam). Alexa Fluor® coupled secondary antibodies were used (Molecular Probes, Eugene, OR, USA). Alexa Fluor® 594 Phalloidin probe were used for F-actin detection (A12381, molecular Probes). CellROX Green probe was used for monitoring intracellular ROS (C10444, molecular Probes). C11-BODIPY (581/591) was used for monitoring lipid peroxidation (C10445, Molecular Probes). TMA-DPH probe was used at 2 µM in membrane fluidity experiments (43060, Sigma, St. Louis, MO, USA).

### 1.3 RNA interference

Ubiad1 siRNA (HSS179099, Invitrogen, Carlsbad, CA, USA) was transfected with lipofectamine RNAiMAX (Invitrogen). After 48 hours, cells were harvested and analyzed by quantitative polymerase chain reaction. As a negative control Stealth RNAi Negative Control Duplexes (Invitrogen) with medium GC content was used as recommended by the manufacturer.

### 1.4 Cell culture

Normal human epithelial keratinocytes were isolated from skin obtained from plastic surgery of healthy females who had given written informed consent. Keratinocytes were cultured in keratinocyte serum free medium, with provided human recombinant epidermal growth factor and bovine pituitary extract (Gibco, Auckland, NZ), and 0.1 mg/ml Primocin™ (Invivogen, San Diego, CA, USA).

### 1.5 Reverse transcription-PCR assays

Total RNA was extracted using mirVana miRNA isolation kit according to the manufacturer's instructions (Ambion, Austin, TX, USA). Total RNA was reverse transcribed with the high capacity cDNA reverse transcription kit (Applied Biosystems, Branchburg, NJ, USA). Real-time polymerase chain reaction was performed according to the Taqman method, using the TaqMan Universal PCR Master Mix (Applied Biosystems) with the StepOnePlus Real-time PCR System (Applied Biosystems). Relative expression levels of each target gene were calculated according to the delta-delta-CT method.

### 1.6 Western blot analysis

Keratinocytes were lysed in RIPA buffer (Thermo Scientific, Rockford, IL, USA), containing protease inhibitor cocktail and EDTA (Thermo Scientific). The protein concentration was determined using the BCA Protein Assay Kit (Thermo Scientific) and equal amounts of total protein were loaded on a 4 to 12% NuPAGE Bis-Tris Gel (Invitrogen) and transferred onto a nitrocellulose membranes using the iBlot dry blotting system (Invitrogen). Non specific binding sites were blocked, and membranes were then labeled with appropriate antibodies and developed using the SuperSignal West Femto Maximum Sensitivity Substrate kit (Thermo Scientific).

### 1.7 Immunofluorescence

Methanol-fixed cells were permeabilized in 0.1% triton X100, blocked in 1% BSA solution. Cells were incubated with primary antibodies diluted in phosphate-buffered saline, followed by incubation with secondary antibodies coupled to Alexa Fluor (Invitrogen). Image acquisition was performed using an Axiovert 200M microscope (Carl Zeiss, Oberkochen, Germany). Photos were captured with an EXI blue camera (Qimaging, Surrey, BC, Canada) coupled to Volocity acquisition software (Perkin Elmer, Waltham, MA, USA). Colocalization of Ubiad1 and Giantin was performed with a statistical approach (17), thus removing the bias of visual interpretation. Pearson correlation coefficient was used as a statistical parameter to quantify the degree of colocalization.

### 1.8 Immunohistological fluorescence

Human skin samples were obtained from plastic surgery of healthy females who had given written informed consent. After removal of subcutaneous fat, tissue was used to obtain 6 mm punch biopsies were taken, fixed in formaldehyde and processed in an automated Shandon Hypercenter XP (Shandon Ltd., Runcor, UK) for paraffin embedding. Sections of 4 µm thickness were cut with a microtome (Shandon) and collected on poly-lysine coated glass slides (Menzel Gläser, Braunschweig, Germany) for immunostaining. Heat and pepsin enzymatic antigen retrieval were performed before incubation with Ubiad1 antibody.

### 1.9 Electron microscopy

Cultured keratinocytes, treated with the peptide at 1 µM concentration for 48 hours were fixed in Karnovsky's solution (Emsdiasum, Hatfield, PA, USA) for 1 h at room temperature, and left for 12 h at 4°C. Cells were then carefully scrapped off the petridish, pelleted with a brief centrifugation, and washed with 0.1 M sodium cacodylate buffer. Pellets were routinely osmicated, dehydrated in a graded series of ethanol, and infiltrated with; and embedded in, a low viscocity epon-epoxy resin. Ultrathin sections of silver-gray interference color were cut and double-stained with uranyl acetate and lead citrate and then examined in a Zeiss 10 transmission electron microscope. Microphotographs were taken with a Gatan digital camera (Gatan, Pleasanton, CA, USA).

### 1.10 Statistical analysis

All experiments have been repeated, the quantification of the fluorescence was performed by measuring the fluorescent intensity of the staining, which was normalized by cell area. Lipid peroxidation in cells was quantified according to manufacturer guideline with the ratios of the signal from the 590 to 510 channels. Statistical analyses were performed using JMP software (SAS, Cary, NC, USA). Normality testing of the data was performed with the Shapiro-Wilk test. The one-way analysis of variance (ANOVA) was used to determine whether there was any significant difference between the means of two or more independent groups. Difference between two means was performed with Student's t-test. A p-value ≤0.05 was considered statistically significant (*), p-value ≤0.01 as very significant (**) and p-value ≤0.005 as highly significant (***).

### 2. Results

### 2.1 Ubiad1 expression and localization in human skin epidermis

Immunostaining for Ubiad1 on human skin sections (Fig. 1A and 1B) showed primary localization of the enzyme in the epidermis, although some cells of the dermis were also positive. Within the epidermis, localization of Ubiad1 was strong in the spinous and basal layers (Fig. 1A), while it was barely detectable in the stratum granulosum. Ubiad1 showed a particular sub-cellular localization as shown by a strong fluorescence aggregation near the nuclei and many cytoplasmic vesicles, while the plasma membrane was virtually unstained (Fig. 1A). Such an asymmetric perinuclear localization strongly suggests that the enzyme is located within cytosolic organelles (Fig. 1B).

### 2.2 Ubiad1 expression and localization within keratinocytes in vitro

Immunofluorescence staining of cultured cells for endogenous Ubiad1 indicated two distinct distribution patterns: first, a punctate staining, and second, a perinuclear localization reminiscent of the Golgi apparatus (Fig. 1C). As displayed in the color overlay images, Ubiad1 showed substantial colocalization with Giantin, a protein that is localized in the Golgi complex. Accordingly, the Pearson correlation coefficient was relatively high (0.79, 0.77); these observations strongly suggest that Ubiad1 associates with the trans-Golgi network (TGN). The Giantin negative, Ubiad1 positive areas are considered to be mitochondrial localization.

### 2.3 Ubiad1 expression decrease in keratinocytes in late passage.

To determine whether Ubiad1 expression decreased during keratinocyte aging, the level of Ubiad1 was measured by immunoblotting in keratinocyte cultured at early and late successive passage. A clearly defined decrease of Ubiad1 expression was observed between early and late cell culture passages *in vitro* (Fig. 2A).

### 2.4 Ubiad1 inducer peptide increases the expression of Ubiad1 in skin keratinocytes

Ubiad1 inducer peptide, i.e. Ala-Val-Leu-Ala-Gly-NH2 (SEQ ID NO: 2), was used to increase the expression of Ubiad1 in skin keratinocytes. As shown in Figs. 2A and 2B, the expression of Ubiad1 in skin keratinocytes was increased by the treatment of the Ubiad1 inducer peptide, data measured by qPCR (+102%) and by immunoblotting (+53%).

### 2.5 Protective effect of Ubiad1 on skin keratinocytes

48 hours after transfection, siRNA-induced silencing of Ubiad1 provided a reduced mRNA expression of 84%. In order to investigate the compensative mechanisms of cells to preserve itself from oxidative stress and subsequent lipid peroxidation, we quantified the expression of Cypla1, one of the most important detoxification enzymes due to its broad substrate specificity. Silencing of Ubiad1 was observed to be associated with a higher expression of Cypla1, suggesting a compensative mechanism to deal with oxidative stress.

Ubiad1 inducer peptide Ala-Val-Leu-Ala-Gly-NH2 (SEQ ID NO: 2) was used to increase the expression of Ubiad1 in skin keratinocytes. As shown in Fig. 2C, transmission electron microscopy of peptide-treated keratinocytes revealed striking differences in cell morphology between the controls and treated. Peptide treatment was associated with a large scale increase in the mitochondrial and Golgi content of the cells, resulting in a highly "activated" state of morphology of the keratinocytes. While mitochondria appeared to have an enlarged and interconnected appearance within the cytosol, the Golgi complex became very prominent, both in numbers of clusters and trans-Golgi elements.

Cumene hydroperoxyde induced a massive ROS accumulation in keratinocytes (Fig. 3A), + 151% compared to control condition (Fig. 3B). Keratinocytes treated with the peptide were observed to be more resistant to ROS accumulation (Figs. 3A and 3B) and lipid peroxidation (Figs. 3C and 3D) companied by the increased expression of Ubiad1, suggesting a role for Ubiad1 modulation in countering oxidative stress.

In addition, Ubiad1 confer protection against SIN-1 peroxynitrite-mediated oxidation, nitrosative stress was evaluated by immunolabeling of 3-nitrotyrosine, a footprint marker of peroxynitrite (ONOO-) and other reactive nitrogen species. Keratinocytes expressing a higher level of Ubiad1 seem to be more resistant to the nitrosative stress (Figs. 3E and 3F).

Keratinocyte membrane permeability was monitored with TMA-DPH (trimethylamine-diphenylhexatriene); a hydrophobic probe exhibiting fluorescence after incorporation into the plasma membrane, cumene hydroperoxide was associated with an observed change in the permeability of the cell membrane (Fig. 3G) (+ 83% of fluorescence intensity compared to control condition) (Fig. 3H). However, such membrane fluidity disruption was not observed in keratinocytes expressing a higher level of Ubiad1 (Fig. 3H).

## Claims

1. Cosmetic use of a composition comprising a peptide of formula (I),
R₁-(AA)ₙ-Ala-Val-Leu-Ala-Gly-(AA)ₚ-R₂ (I)
wherein
AA represents any amino acid or one of its derivatives, and n and p are integers between 0 and 4;
R₁ represents the primary amine function of the N-terminal amino acid, free or substituted by a protector group which can be selected from acetyl, benzyl, tosyl and benzyloxycarbonyl;
R₂ represents the hydroxyl function of the carboxyl acid of C-terminal amino acid, free or substituted by a protector group which can be selected from C₁ to C₃₀ alkyl, -NH₂, -NHY and -NYY group with Y representing a C₁ to C₄ alkyl, for increasing the expression of Ubiad1 in skin, skin appendages or epidermal cells including keratinocytes for improving physiological functions of the skin, comprising contacting the skin, skin appendages or epidermal cells with a composition containing the peptide, wherein the peptide of formula (I) consists of the amino acid sequence of SEQ ID NO:1 or SEQ ID NO:2.

2. The cosmetic use of claim 1, wherein the cells are epidermal keratinocytes.

3. The cosmetic use of anyone of claims 1 or 2, wherein the skin is epidermis tissues.

4. The cosmetic use of claim 3, wherein epidermis tissues are spinous layer and basal layer of the epidermis.

## Patentansprüche

1. Kosmetische Verwendung einer Zusammensetzung umfassend ein Peptid mit der Formel (I),
R₁-(AA)ₙ-Ala-Val-Leu-Ala-Gly-(AA)ₚ-R₂ (I)
wobei
AA eine beliebige Aminosäure oder eines ihrer Derivate darstellt und n und p ganze Zahlen zwischen 0 und 4 sind;
R₁ die primäre Aminfunktion der N-terminalen Aminosäure darstellt, frei oder substituiert durch eine Schutzgruppe, die aus Acetyl, Benzyl, Tosyl und Benzyloxycarbonyl ausgewählt sein kann;
R₂ die Hydroxylfunktion der Carbonsäure der C-terminalen Aminosäure darstellt, frei oder substituiert durch eine Schutzgruppe, die aus einem C₁- bis C₃₀-Alkyl, einer -NH₂-, -NHY- und - NYY-Gruppe, bei der Y ein C₁- bis C₄-Alkyl darstellt, ausgewählt sein kann,
zur Erhöhung der Expression von Ubiad1 in der Haut, in Hautanhangsgebilden oder epidermalen Zellen, einschließlich Keratinozyten, zur Verbesserung physiologischer Funktionen der Haut, umfassend das Inberührungbringen der Haut, Hautanhangsgebilde oder epidermalen Zellen mit einer das Peptid enthaltenden Zusammensetzung, und
wobei das Peptid mit der Formel (I) aus der Aminosäuresequenz SEQ ID Nr: 1 oder SEQ ID Nr: 2 besteht.

2. Kosmetische Verwendung nach Anspruch 1, wobei es sich bei den Zellen um epidermale Keratinozyten handelt.

3. Kosmetische Verwendung nach einem der Ansprüche 1 oder 2, wobei es sich bei der Haut um Epidermisgewebe handelt.

4. Kosmetische Verwendung nach Anspruch 3, wobei es sich bei den Epidermisgeweben um die Stachelzellschicht und die Basalzellschicht der Epidermis handelt.

## Revendications

1. Utilisation cosmétique d'une composition comprenant un peptide de formule (I),
R₁-(AA)ₙ-Ala-Val-Leu-Ala-Gly-(AA)ₚ-R₂ (I)
Dans laquelle
AA représente un acide aminé quelconque, ou un de ses dérivés, et n et p sont des nombres entiers compris entre 0 et 4 ;
R₁ représente la fonction amine primaire de l'acide aminé N-terminal, libre ou substituée par un groupement protecteur pouvant être sélectionné parmi un acétyle, un benzyle, un tosyle ou un benzyloxycarbonyle ;
R₂ représente la fonction hydroxyle de l'acide carboxylique de l'acide aminé C-terminal, libre ou substituée par un groupement protecteur pouvant être sélectionné parmi un alkyle de C₁ à C₃₀, un -NH₂, un -NHY ou -NYY dans lesquels Y représente un alkyle de C₁ à C₄,
Pour augmenter l'expression de Ubiad1 dans la peau, les phanères ou les cellules épidermiques incluant les kératinocytes, pour améliorer les fonctions physiologiques de la peau, comprenant la mise en contact de la peau, des phanères ou des cellules épidermiques avec une composition contenant le peptide, et
dans laquelle le peptide de formule (I) consiste en la séquence d'acides aminés SEQ ID No:1 ou SEQ ID No:2.

2. Utilisation cosmétique selon la revendication 1, dans laquelle les cellules sont des kératinocytes épidermiques.

3. Utilisation cosmétique selon l'une des revendications 1 ou 2, dans laquelle la peau est des tissus épidermiques.

4. Utilisation cosmétique selon la revendication 3, dans laquelle les tissus épidermiques sont la couche épineuse et la couche basale de l'épiderme.


### SEQUENCE LISTING

<110> ISP INVESTMENTS LLC
<120> COSMETIC USE AND METHOD FOR IMPROVING PHYSIOLOGICAL FUNCTIONS OF THE SKIN BY INCREASING THE EXPRESSION OF UBIAD1
<130> NTD128185
<160> 2
<170> PatentIn version 3.5
<210> 1
   <211> 5
   <212> PRT
   <213> Artificial Sequence
<220>
   <223> Synthetic peptide
<400> 1
   Ala Val Leu Ala Gly
   1               5
<210> 2
   <211> 5
   <212> PRT
   <213> Artificial Sequence
<220>
   <223> Synthetic peptide
<220>
   <221> MOD_RES
   <222> (5)..(5)
   <223> AMIDATION
<400> 2
   Ala Val Leu Ala Gly
   1               5